# EUROPEAN PATENT SPECIFICATION

(11) **EP 0 622 060 B1**
(45) Date of publication and mention of the grant of the patent: **20.09.2000**
(21) Application number: 93303377.1
(22) Date of filing: 29.04.1993
(51) Int. Cl.: A61F 2/24, A61L 27/00

(54) **Improvement in or relating to prosthetic cardiac valve and to the method of manufacturing same**
Verbesserte Herzklappe und ihr Herstellungsverfahren
Valve cardiaque améliorée et son procédé de fabrication

(43) Date of publication of application: 02.11.1994
(73) Proprietor: NATIONAL RESEARCH DEVELOPMENT CORPORATION, New Delhi 110 048 (IN)
(72) Inventor: Bhuvaneshwar, Gobichettipalayam Subbaratnam, Poojapura, Trivandrum 695 012, Kerala (IN); Vayalappil, Muraleedharan Chirathody, Poojapura, Trivandrum 695 012, Kerala (IN); Nair, Omana Amma Sreedharan, Poojapura, Trivandrum 695 012, Kerala (IN)
(74) Representative: Simpson, Alison Elizabeth Fraser

(56) References cited:
- EP-A- 0 145 884
- EP-A- 0 376 503
- WO-A-84/00882
- DE-A- 2 908 222
- US-A- 3 824 629
- US-A- 4 532 659
- US-A- 4 556 996

## Description

This invention relates to improvements in or relating to prosthetic cardiac valve and to the method of manufacturing same.

It is already known to have several types of artificial heart valves all of which generally operate due to the continuous pumping action of the heart. These heart valves usually function as check valves ensuring unidirectional flow of blood.

This invention more particularly relates to improvements in or relating to artificial heart valves which have a plate or disc mounted tiltably in a cage, which cage has an outer ring of a sewing component adapted to be secured inside the heart where required. Bileaflet heart valves are also in vogue of several constructions like single plate heart valves.

Since heart valves are to function continuously and the disc which is tiltably mounted has to flutter more than 1, 15,000 times per day in a normal person having 72 to 80 beats per minute, the artificial disc used has to have long operational life, abrasion and wear resistance and should also maintain the same clerance between it and the valve housing for a long time so that replacement is not necessary. Once installed, the heart valve has to function flawlessly for years together and, therefore, it is most important to have proper choice of the materials of the various components and optimize their construction. There are a number of Patents already granted and a considerable amount of literature is also available on heart valves including the Applicants own earlier U.S. Patent No.4,822,355 (Ind. Pat. 159244, 167706). Several other U.S. Patents of relevance that have already been studied by the Applicant include U.S. Patent No.4,532,659, U.S. Patent No.4,687,487, U.S. Pat. No.4,263,680, U.S. Pat. No.2,947,716, U.S. Pat. No. 4,057,857, U.S. Pat. No.4,494,253, U.S. Pat. No.3,959,827, U.S. Pat. No.3.824,629, U.S. Pat. No.3698018, U.S.Pat No. 3737919, U.S. Pat. No.383547 5, U. S. Patent No.5,026,391 and German Patent DE-A. 3504962 and several other literature.

As already stated there are many types of mechanical heart valve prostheses currently in use, viz. caged ball valves (E.g. Starr - Edwards ball valve), tilting disc valves (e.g. Bjork-Shiley valve, Medtronic - Hall valve) and bileaflet valves (St. Jude Medical Valve).

The disc of a tilting disc valve has to open and close 40 million times a year at an average, causing tremendous strain on the material. So the material used should be extremely durable in terms of wear resistance and fatigue strength. A valve may have to operate inside the human body up to 25 years or more which necessitates the material to have excellent chemical and structural stability. The biocompatibility (both tissue compatibility and blood compatibility) of the material is also a major requirement to ensure acceptability of the implant by the body. Finally the material should be processable to achieve the required surface finish and dimensional stability.

Towards achieving the above objectives, many researchers have come out with several suggestions both patented and otherwise published.

Thus, although many polymer materials were suggested for use in prosthetic heart valves, only silicon rubber (used in Starr - Edwards ball Valves) has gained acceptability among them. Polyethylenes are another family of materials suggested by other groups. But all forms of Polyethylenes are not suitable for using as disc material in valves.

The intensive study by the Applicant has shown that Low Density Poly Ethylene (LDPE) does not have the required wear resistance and load bearing capacity to be used for this application. High Density Poly ethylene (HDPE), being more amorphous in nature, exhibits lower toughness, fatigue strength and decreased stress crack resistance.

It is thus a field open for further research and development and the search is still on for a near ideal material.

It has been found by the Applicant that a modified form of Ultra High Molecular Weight Poly Ethylene (UHMW-PE) is best suited for the purposes of heart valve applications.

As such, UHMW - PE is being used in certain orthopedic implants (especially as acetabular cups in total hip joints) and has excellent track record as a load bearing material, in these applications.

UHMW-PE exhibits excellent tissue and blood compatibility and is stable (both chemically and structurally) for long duration of working in physiological environments. It has extremely good wear resistance, fatigue strength and toughness.

However, due to the poor melt flow properties of UHMW-PE, it is difficult to prepare heart valve discs/plates out of UHMW - PE, to achieve the desired high degree of surface finish.

It is very important that heart valve discs possess a very high degree of surface finish so that the tendency of clot formation of the flowing blood on the disc surface is subtantially eliminated. It is required to have a surface finish of the order of 0.1 micron or better.

Due to the high melt flow index, particles of UHMW-PE do not melt and fuse uniformly during conventional compression moulding using resin powder. This produces local regions of poorly fused UHMW PE which are susceptible to high rates of degradation.

It is, therefore, an object of this invention to propose improved heart valve disc using modified UHMW-PE which will ensure moulded heart valve disc which can be given high surface finish and combine all the other advantageous properties such as durability, logevity, high stress resistance, wear resistance, blood compatability, etc.

It is another object of this invention to propose a method for preparing modified UHMW-PE suitable for the manufacture of improved heart valve disc.

It is a further object of this invention to propose a carefully controlled method of manufacturing improved heart valve disc from modified UHMW-PE WHICH METHOD IS DEPENDABLE AND REPRODUCIBLE.

In short, the method of preparing the heart valve disc involves a Solid State Compaction technique for generating the required surface finish along with a Cryo-Machining technique for flash removal.

Thus, according to this invention there is provided a method for the preparation of improved heart valve disc from polyethylene which comprises:
(i) subjecting a ram extruded UHMW - PE rod of suitable size to machining under Cryo-machining conditions at temperatures below O°C to obtain a blank corresponding to the shape of the heart valve disc;
(ii) subjecting the blank obtained in step (i) above to a solid state compaction step at temperatures not exceeding 150°C and at forces not exceeding 30 kN, in a suitable die, to obtain compacted disc of the required profile and corresponding to the die used;
(iii) annealing the compacted disc in a water bath to relieve the disc of any thermally generated stress in step (ii) above;
(iv) subjecting the annealed disc to a step of edge trimming under cryo-machining conditions to remove any thin flashes produced on the edge of the disc in step (i) above;
(v) followed by a further annealing step in water to relieve from the formed disc any thermal stress produced in step (iv); and
(vi) finally subjecting the so formed disc to a step of ultrasonic cleaning to remove any surface contamination.

Further, according to this invention there is provided an improved heart valve assembly comprising a sewing ring component, mounted on the outer circumference of a valve housing component, a disc occluder mounted within the inner circumference of the valve housing, said disc occluder being held tiltably on supports within the enclosed space of the valve housing component, said valve housing component having a grooved ring portion externally on which is mounted said sewing ring component, the sewing ring component being fabricated from polyester, the valve housing component being fabricated of a cobalt - nickel based or cobalt-chromium tungsten based alloy or titanium and its alloys, wherein the valve disc is fabricated from PE,
characterised in that said PE from which said valve disc is fabricated is UHMW-PE modified according to the above method.
(a) The disc annealing is done for a suitable duration between 1 to 6 hrs at a temperature below 120°C.
(b) The cooling rate is less than 1°C per minute.

The ram extruded UHMW-PE rod is subjected to cryo-machining at temperatures below 0°C.

The cryo-machining is carried out in the presence of anti-freeze and anti - rust coolant.

The anti - freeze and anti-rust coolant is a mixture or polypropylene glycol and water. Though a range of ratios of these two liquids can be used, it is advantageous to use a mixture having polypropylene glycol and water in the ratio of 1:1.5 by volume.

For optimum results and to prevent corrosion, the said coolant also includes inorganic salts such as potassium dichromate, sodium nitrate, sodium bicarbonate and borax in suitable weight percentages/ratios so that the pH of the medium is around 8.5. The pH can vary slightly either way.

## Claims

1. A method for the preparation of improved heart valve disc from polyethylene which comprises;
(i) subjecting a ram extruded UHMW-PE rod of suitable size to machining under Cryo-machining conditions at temperatures below 0°C to obtain a blank corresponding to the shape of the heart valve disc;
(ii) subjecting the blank obtained in step (i) above to a solid state compaction step at temperatures not exceeding 150°C and at forces not exceeding 30 kN, in a suitable die, to obtain compacted disc of the required profile and corresponding to the die used;
(iii) annealing the compacted disc in a water bath to relieve the disc of any thermally generated stress in step (ii) above;
(iv) subjecting the annealed disc to a step of edge trimming under cryo-machining conditions to remove any thin flashes produced on the edge of the disc in step (i) above;
(v) followed by a further annealing step in water to relieve from the formed disc any thermal stress produced in step (iv); and ;
(vi) finally subjecting the so formed disc to a step of ultrasonic cleaning to remove any surface contamination.

2. A method as claimed in claim 1 wherein the disc annealing is done for a suitable duration between 1 to 6 hrs at a temperature below 120°C.

3. A method as claimed in claim 2 wherein the cooling of annealed disc is carried out by cooling at a rate of less than 1°C per minute.

4. A method as claimed in claim 1 wherein the ram extruded UHMW-PE rod is subjected to cryo-machining at temperatures below 0°C.

5. A method as claimed in claims 1 or 4, wherein the cryo-machining is carried out in the presence of anti-freeze and anti-rust coolant.

6. A method as claimed in claim 5 wherein the anti-freeze and anti-rust coolant is a mixture of polypropylene glycol and water.

7. A method as claimed in claim 6 wherein said mixture comprises polypropylene glycol and water in the ratio of 1:1.5 by volume.

8. A method as claimed in claim 5 wherein the said coolant also includes inorganic salts such as potassium di-chromate, sodium nitrate, sodium bicarbonate and borax in suitable weight percentages/ratios so that the pH of the medium is around 8.5.

9. A method as claimed in any of the preceding claims wherein the valve disc is one which has been simultaneously fabricated and modified.

10. An improved heart valve assembly comprising a sewing ring component, mounted on the outer circumference of a valve housing component, a disc occluder mounted within the inner circumference of the valve housing, said disc occluder being held tiltably on supports within the enclosed space of the valve housing component, said valve housing component having a grooved ring portion externally on which is mounted said sewing ring component, the sewing ring component being fabricated from polyester, the valve housing component being fabricated of a cobalt - nickel based or cobalt-chromium tungsten based alloy or titanium and its alloys, wherein the valve disc is fabricated from PE,
characterized in that said PE from which said valve disc is fabricated is UHMW-PE modified according to the method of any one of the preceding claims.

## Patentansprüche

1. Verfahren zur Herstellung von verbesserten Herzklappen-Scheiben aus Polyethylen, umfassend:
(i) das Unterziehen eines sinterextrudierten UHMW-PE-Stabs geeigneter Größe einer Bearbeitung unter Kryobearbeitungsbedingungen bei Temperaturen unter 0°C zur Erzielung eines der Gestalt der Herzklappen-Scheibe entsprechenden Rohlings;
(ii) das Unterziehen des im obigen Schritt (i) erhaltenen Rohlings einem Festkörperverdichtungsschritt bei Temperaturen, die 150°C nicht übersteigen, und mit einer Kraft, die 30 kN nicht übersteigt, in einer geeigneten Form zur Erzielung einer verdichteten Scheibe gewünschten Profils entsprechend der verwendeten Form;
(iii) das Abkühlen der verdichteten Scheibe in einem Wasserbad zum Befreien der Scheibe von jeglicher im obigen Schritt (ii) thermisch erzeugter Spannung;
(iv) das Unterziehen der abgekühlten Scheibe einem Kantenbeschneidungsschritt unter Kryobearbeitungsbedingungen zur Entfernung jeglicher im obigen Schritt (i) am Rand der Scheibe erzeugter dünner Grate;
(v) anschließend einen weiteren Abkühlschritt in Wasser zum Befreien der geformten Scheibe von jeglicher im obigen Schritt (iv) erzeugter thermischer Spannung; und
(vi) zuletzt das Unterziehen der so geformten Scheibe einem Schritt der Ultraschallreinigung zur Entfernung jeglicher Oberflächenverunreinigung.

2. Verfahren nach Anspruch 1, worin das Abkühlen der Scheibe über einen zweckmäßigen Zeitraum von 1 bis 6 Stunden bei einer Temperatur unter 120°C erfolgt.

3. Verfahren nach Anspruch 2, worin das Kühlen der abgekühlten Scheibe durch Kühlen mit einer Geschwindigkeit von weniger als 1°C pro Minute erfolgt.

4. Verfahren nach Anspruch 1, worin der sinterextrudierte UHMW-PE-Stab einer Kryobearbeitung bei Temperaturen unter 0°C unterzogen wird.

5. Verfahren nach Anspruch 1 oder 4, worin die Kryobearbeitung in Gegenwart eines nicht frierenden und nicht rostenden Kühlmittels durchgeführt wird.

6. Verfahren nach Anspruch 5, worin das nicht frierende und nicht rostende Kühlmittel eine Mischung aus Polypropylenglycol und Wasser ist.

7. Verfahren nach Anspruch 6, worin die Mischung Polypropylenglycol und Wasser in einem Volumenverhältnis von 1:1,5 enthält.

8. Verfahren nach Anspruch 5, worin das Kühlmittel auch anorganische Salze wie Kaliumdichromat, Natriumnitrat, Natriumbicarbonat und Borax in geeigneten Gewichtsprozent/-verhältnissen enthält, sodass der pH-Wert des Mediums etwa 8,5 beträgt.

9. Verfahren nach einem der vorhergehenden Ansprüche, worin die Klappenscheibe eine Scheibe ist, die gleichzeitig gefertigt und modifiziert worden ist.

10. Verbessertes Herzklappensystem umfassend einen am Außenumfang eines Klappengehäuseteils gelagerten Heftringteil und eine innerhalb des Innenumfangs des Klappengehäuses gelagerte Scheibenschließeinrichtung, welche Scheibenschließeinrichtung auf Trägern innerhalb des umschlossenen Raums des Klappengehäuseteils kippbar gehalten ist, wobei der Klappengehäuseteil außen einen Ringabschnitt mit Nut aufweist, auf welchem der Heftringteil gelagert ist, wobei der Heftringteil aus Polyester gefertigt ist, der Klappengehäuseteil aus einer Legierung auf Kobalt-Nickel-Basis oder Kobalt-Chrom-Wolfram-Basis oder aus Titan oder Legierungen davon gefertigt ist und die Klappenscheibe aus PE gefertigt ist, dadurch gekennzeichnet, dass das PE, aus dem die Klappenscheibe gefertigt ist, ein nach dem Verfahren gemäß einem der vorhergehenden Ansprüche modifiziertes UHMW-PE ist.

## Revendications

1. Procédé pour préparer un disque de valve cardiaque amélioré à partir de polyéthylène, qui comprend les étapes suivantes :
(i) soumettre une tige de PEhpm extrudé par plongeur d'une dimension appropriée à un usinage dans des conditions d'usinage cryogénique à des températures inférieures à 0°C pour obtenir une ébauche correspondant à la forme du disque de valve cardiaque ;
(ii) soumettre l'ébauche obtenue dans l'étape (i) ci-dessus à une étape de compactage à l'état solide à des températures n'excédant pas 150°C et sous des forces n'excédant pas 30 kN, dans une matrice appropriée, pour obtenir un disque compacté du profil recherché et correspondant à la matrice utilisée ;
(iii) soumettre le disque compacté à un chauffage de revenu dans un bain d'eau pour libérer le disque de toutes les contraintes générées par la chaleur dans l'étape (ii) ci-dessus ;
(iv) soumettre le disque ainsi revenu à une étape de dressage du bord dans des conditions d'usinage cryogénique pour enlever toutes les minces bavures produites sur le bord du disque dans l'étape (i) ci-dessus ;
(v) soumettre à une nouvelle étape de chauffage de revenu dans l'eau le disque ainsi traité pour libérer le disque formé de toute contrainte thermique produite dans l'étape (iv) ; et
(vi) soumettre finalement le disque ainsi formé à une étape de nettoyage ultrasonique pour enlever toute contamination de surface.

2. Procédé selon la revendication 1, dans lequel le revenu du disque est effectué pendant une durée appropriée comprise entre 1 et 6 heures et sous une température inférieure à 120°C.

3. Procédé selon la revendication 2, dans lequel le refroidissement du disque revenu est effectué par un refroidissement à un gradient inférieur à 1°C par minute.

4. Procédé selon la revendication 1, dans lequel la tige de PEhpm extrudé par plongeur est soumise à un usinage cryogénique à une température inférieure à 0°C.

5. Procédé selon le revendication 1 et/ou la revendication 4, dans lequel l'usinage cryogénique est effectué an présence d'un agent de refroidissement anti-congélation et anti-rouille.

6. Procédé selon la revendication 5, dans lequel l'agent de refroidissement anti-congélation et anti-rouille est un mélange de polypropylène glycol et d'eau.

7. Procédé selon la revendication 6, dans lequel ledit mélange comprend du polypropylène glycol et de l'eau en un rapport volumétrique de 1:1,5.

8. Procédé selon la revendication 5, dans lequel ledit agent de refroidissement contient également des sels inorganiques tels que dichromate de potassium, nitrate de sodium, bicarbonate de sodium et borax en des pourcentages ou rapports en poids appropriés de façon que le pH du milieu soit voisin de 8,5.

9. Procédé selon l'une des revendications précédentes, dans lequel le disque de valve est un disque qui a été simultanément fabriqué et modifié.

10. Ensemble de valve cardiaque amélioré, comprenant un anneau à coudre, monté sur la circonférence extérieure d'un logement de valve, un disque d'occlusion monté à l'intérieur de la circonférence intérieure du logement de valve, ledit disque d'occlusion étant maintenu, basculant, sur des supports à l'intérieur de l'espace clos du logement de valve, ledit logement de valve ayant une portion d'anneau rainuré extérieur sur lequel est monté ledit anneau à coudre, l'anneau à coudre étant fabriqué en polyester, le logement de valve étant fabriqué en un alliage à base de cobalt et de nickel ou à base de cobalt, de chrome et de tungstène, ou en titane et ses alliages, dans lequel le disque de valve est fabriqué an polyéthylène,
caractérisé en ce que ledit polyéthylène dont est fabriqué le disque de valve est un PEhpm modifié selon le procédé selon l'une quelconque des revendications précédentes.
